# EUROPÄISCHE PATENTSCHRIFT

(11) **EP 0 371 908 B1**
(45) Veröffentlichungstag und Bekanntmachung des Hinweises auf die Patenterteilung: **26.01.1994**
(21) Anmeldenummer: 89730209.7
(22) Anmeldetag: 26.10.1989
(51) Int. Cl.: A61L 27/00, A61N 1/375

(54) **Implantat**
Implant
Implant

(30) Priorität: 29.11.1988 DE 8815083 U
(43) Veröffentlichungstag der Anmeldung: 06.06.1990
(73) Patentinhaber: BIOTRONIK Mess- und Therapiegeräte GmbH & Co Ingenieurbüro Berlin, D-12359 Berlin (DE)
(72) Erfinder: Bolz, Armin, Dipl.-Phys., D-8520 Erlangen (DE); Schaldach, Max, Prof. Dr.-Ing., D-8520 Erlangen (DE)
(74) Vertreter: Christiansen, Henning, Dipl.-Ing.

(56) Entgegenhaltungen:
- EP-B- 0 029 787
- DD-A- 133 518
- DD-A- 229 028
- DE-A- 2 613 052
- DE-A- 3 300 672
- DE-A- 3 516 411
- DE-C- 3 116 040
- FR-A- 2 566 272
- DERWENT ACCESSION NO. 87-194 481, Questel Tele- systems (WPIL) DERWENT PUBLICATIONS LTD., London

## Beschreibung

Die Erfindung betrifft ein Implantat der im Oberbegriff des Anspruchs 1 angegebenen Art.

Derartige kardiovaskuläre Implantate werden vorzugsweise als Herzklappenprothesen oder Herzschrittnacherelektroden im menschlichen Körper eingesetzt. Die Blutverträglichkeit kordiovaskulärer Implantate wird jedoch entscheidend von der Beschaffenheit ihrer Oberfläche beeinflußt. Für die Antithrombogenität ist zum einen eine geringe Rauhtiefe notwendig um die Anlagerung bzw. Zerstörung korpuskulärer Bestandteile des Blutes und die damit verbundene Aktivierung des Gerinnungssystemes zu verhindern. Desweiteren müssen jedoch auch direkte Ladungsaustauschprozesse zwischen gerinnungsspezifischen Proteinen und der Implantatoberfläche verhindert werden.

Aufgrund dieser spezifischen Anforderungen sind für den breiten Einsatz als Implantatwerkstoffe z.B. in EP-B1-0 029 787 und DD-A-133 518 vorgeschlagene Schichtstrukturen, etwa mit verschiedenen Aluminiumverbindungen oder Siliziumnitrid/Kalziumfluorid, für kardiovaskuläre Implantate wenig gebräuchlich geworden.

Es ist bekannt, zur Erfüllung dieser Erfordernisse Beschichtungen aus pyrolytischem Kohlenstoff als dem gebräuchlichsten Werkstoff für Herzklappen einzusetzen. Dies ist beispielsweise in der DE-A1-31 16 040 beschrieben. Daneben ist er bekannt, zur Verhinderung der Ladungsaustauschprozesse zwischen den gerinnungsspezifischen Proteinen und der Implantatoberfläche halbleitende Materialien, vorzugsweise Rutilkeramik, als Implantatwerkstoff zu verwenden.

Diese Art der Implantate mit einer Beschichtung aus Kohlenstoff mit seiner porösen Struktur werden zwar den Ansprüchen an die Oberflächenbeschaffenheit gerecht, sie haben jedoch den Nachteil, daß ein Elektronentransfer durch Tunneln von besetzten, valenzbandartigen Zuständen des Proteins in freie Zustände des Festkörpers zu einer Spaltung des Fibrinogens im Blut führt. Die dabei entstehenden Fibrinmonomere können sich polymerisieren und zu einem irreversiblen Thrombus führen. Implantate aus Rutilkeramik unterbinden auch diese Ladungsaustauschprozesse, sie sind jedoch aufgrund der hohen Produktionskosten nicht zur Serienreife gelangt.

Weiterhin ist in der Derwent Accession No. 87-194 481 ein Implantat beschrieben, welches unter anderem mit einer Oberflächenbeschichtung aus Siliziumkarbid, Siliziumnitrid oder Siliziumoxid versehen sein kann. Die Beschichtungen erhöhen die Blutverträglichkeit, Festigkeit und die Korrisionsbeständigkeit des Implantats.

Der Erfindung liegt die Aufgabe zugrunde, bei einem Implantat der eingangs genannten Gattung eine kardiovaskulär verträgliche Oberfläche zu schaffen, die auch kostengünstig herzustellen ist.

Diese Aufgabe wird mit den kennzeichnenden Merkmalen des Anspruchs 1 gelöst.

Die Verwendung einer Beschichtung aus einem der halbleitenden Materialien Siliziumkarbid, Siliziumnitrid oder Siliziumoxid, welches mit Phosphor dotiert worden ist, ist bei einem Implantat deswegen besonders vorteilhaft, weil mit einer äußerst dünnen Oberflächenschicht aus Halbleitermaterial Elektronenströme, welche eine Fibrinaktivierung begünstigen, verhindert werden.

Die entsprechenden Beschichtungen lassen sich kostengünstig durch PVD- oder CVD-Verfahren erzeugen, wobei zur Phosphordotierung der Phospher in Form von Phosphin (PH₃) in einer Konzentration von unter einem Prozent eingebracht wird. Dabei ist insbesondere die Erzeugung von amorphem SiC nach dem sogenannten Plasma-CVD-Verfahren (Plasma assisted chemical vapor deposition) günstig. Auf diese Weise lassen sich Schichten sehr geringer elektrischer Zustandsdichte erzeugen.

Insbesondere sind noch folgende vorteilhafte Weiterbildungen günstig:
Die porenfreie Halbleiterbeschichtung besteht entweder aus einem amorphen oder einem mikrokristallinen Werkstoff. Die Rauhtiefe der Oberfläche beträgt weniger als 0,1 µm. Dadurch wird die Anlagerung bzw. Zerstörung korpuskulärer Bestandteile des Blutes und die damit verbundene Aktivierung des Gerinnungssystemes verhindert.

Die Lösung basiert auf der Erkenntnis, daß auf dem energetischen Niveau der elektronischen Proteinzustände die Zustandsdichte der Beschichtung gering ist. Damit werden thrombogene Ladungsaustauschprozesse zwischen gerinnungsspezifischen Proteinen und der Implantatoberfläche verhindert.

Bevorzugt wird zur antithrombogenen Beschichtung amorphes Siliziumkarbid (a-SiC:H), ein Abscheidungsprodukt eines Gemisches enthaltend Silan (SiH₄) und/oder Methan (CH₄), verwendet.

Die Beschichtung ist insbesondere möglichst porenfrei ausgebildet. Dadurch erhöht sich auch deren Stabilität. Statt Siliziumkarbid kann auch Siliziumnitrid (a-SiN:H) benutzt werden, das insoweit übereinstimmende Oberflächeneigenschaften aufweist.

Um die Haftfähigkeit zwischen Substrat und Beschichtung zu verbessern und insbesondere zu verhindern, daß sich beim Abkühlen nach dem Beschichtungsprozeß Teile der Beschichtung ablösen, ist es günstig, zwischen Substrat und Beschichtung eine Zwischenschicht vorgesehen ist, welche Wärmedehnungen ausgleicht, wobei dieser Ausgleich durch einen Werkstoff mit wesentlich geringerem Elastizitätsmodul erfolgt.

Bevorzugt besteht diese Zwischenschicht aus Graphit, Glaskohlenstoff oder pyrolitischem Kohlenstoff bzw. bildet bei einem metallischen Substrat eine Legierung der Materialien von Beschichtung und Substrat.

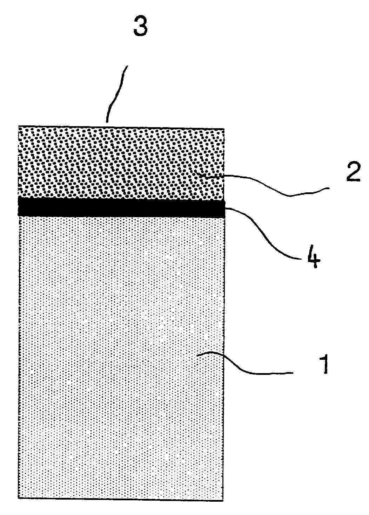

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figur näher dargestellt.

Die einzige Figur zeigt ein Ausführungsbeispiel der Erfindung als Schnitt durch eine Oberflächenschicht des Implantats.

Bei dem in der Figur dargestellten Ausführungsbeispiel eines erfindungsgemäßen kardiovaskulären Implantats ist dessen Schichtaufbau im Querschnitt wiedergegeben. Auf der Oberfläche eines Implantats, gebildet durch ein aus einem Grundwerkstoff bestehendes Teil 1, befindet sich eine porenfreie Beschichtung 2 aus amorphem Siliziumkarbid (a-SiC:H) als Halbleiterwerkstoff, das ein Abscheidungsprodukt eines Gemisches enthaltend Silan (SiH₄) und/oder Methan (CH₄) bildet und dessen Oberfläche antithrombogen ausgebildet ist. Bei dem Teil 1 handelt es sich beispielsweise um den äußeren Ring einer Herzklappenprothese oder die nicht isolierten (aktiven) Bereiche einer Herzschrittmacherelektrode.

Die Dicke der Beschichtung 2 liegt insbesondere im Bereich von bis zu 1 µ und beträgt maximal 10 µm. Sie ist weicher und hat einen wesentlich geringeren Elastizitätsmodul als die benachbarten Schichten.

Die Oberfläche 3 weist eine Rauhtiefe von ca. 0,1 µm oder weniger auf. Die Zustandsdichte der Beschichtung 2 liegt im Bereich des energetischen Niveaus der Proteinzustände im Blut, also bei 10¹⁸ cm⁻³eV-1 oder niedriger. Die Ausbildung der Oberfläche 3 verhindert direkte Ladungsaustauschprozesse zwischen gerinnungsspezifischen Proteinen und der Implantatoberfläche.

Bei einer weiteren Ausführungsvariante befindet sich auf einem aus einem Grundwerkstoff bestehenden Teil 1 eine Schicht 2 aus einem mikrokristallinen Halbleiterwerkstoff, insbesondere Siliziumnitrid (a-SiN:H). Im übrigen weist die Oberfläche 3 dieses Ausführungsbeispiels entsprechende Eigenschaften wie die des zuvor beschriebenen Ausführungsbeispiels auf.

Zur Stabilisierung des Silikatnetzwerkes ist insbesondere eine geringfügige Dotierung mit Phosphor vorgesehen, insbesondere in Form von Phosphin mit einer Konzentration von weniger als einem Prozent. Hiermit ist gleichzeitig die erwünschte Erhöhung der Leitfähigkeit verbunden.

Bei dem in der Figur ausschnittsweise dargestellten Implantat ist zwischen Substrat und Beschichtung eine Zwischenschicht 4 vorgesehen, welche Wärmedehnungen ausgleicht. Dieser Ausgleich erfolgt durch eine geringere Dichte und/oder einen thermischen Ausdehnungskoeffizienten der Zwischenschicht, dessen Wert zwischen demjenigen des Substrats und der Beschichtung liegt. Während eine Beschichtung geringerer mechanischer Dichte die thermischen Ausgleichsbewegungen elastisch "puffert", bewirkt ein Zwischenwert des thermischen Ausdehnungskoeffizienten einen Ausgleich mechanischer Spannungen, der die Haftfestigkeit verbessert. Die Zwischenschicht 4 besteht bei dem dargestellten Ausführungsbeispiel aus Graphit, Glaskohlenstoff oder pyrolitischem Kohlenstoff. Bei einem metallischen Substrat würde die Zwischenschicht 4 bevorzugt eine Legierung der Materialien von Beschichtung und Substrat bilden.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Patentansprüche

1. Implantat, insbesondere für kardiovaskuläre Anwendung, mit einer Beschichtung (2), die aus einem der halbleitenden Materialien Siliziumkarbid (a-SiC:H), Siliziumnitrid (a-SiN:H) oder Siliziumoxid (a-SiO:H) besteht,
**dadurch gekennzeichnet,**
daß zur Stabilisierung des Silikatnetzwerkes eine geringfügige Dotierung der Beschichtung (2) mit Phosphor vorgesehen ist, insbesondere in Form von Phosphin mit einer Konzentration von weniger als einem Prozent.

2. Implantat nach Anspruch 1, **dadurch gekennzeichnet,** daß das halbleitende Material amorph ist.

3. Implantat nach Anspruch 1, **dadurch gekennzeichnet,** daß das halbleitende Material mikrokristallin ist.

4. Implantat nach einem der vorangehenden Ansprüche, **dadurch gekennzeichnet,** daß die Beschichtung (2) eine Dicke im Bereich von 1 µm, maximal 10 µm, aufweist.

5. Implantat nach Anspruch 4, **dadurch gekennzeichnet,** daß die Oberflächenrauhigkeit im Bereich von 0,1 µm liegt.

6. Implantat nach einem der vorangehenden Ansprüche, **dadurch gekennzeichnet,** daß die Zustandsdichte der Beschichtung (2) bei 10¹⁸cm⁻³eV⁻¹ oder niedriger liegt, so daß direkte Ladungsaustauschprozesse zwischen gerinnungsspezifischen Proteinen und der Implantatoberfläche (3) verhindert werden.

7. Implantat nach Anspruch 2, **dadurch gekennzeichnet,** daß das amorphe Siliziumkarbid (a-SiC:H) insbesondere ein Abscheidungsprodukt eines Gemisches, enthaltend Silan (SiH4) und/oder Methan (CH4), ist.

8. Implantat nach einem der vorangehenden Ansprüche, **dadurch gekennzeichnet,** daß die Beschichtung (2) im wesentlichen porenfrei ist.

9. Implantat nach einem der vorangehenden Ansprüche, **dadurch gekennzeichnet,** daß zwischen Substrat und Beschichtung (2) eine Zwischenschicht (4) vorgesehen ist, welche Wärmedehnungen ausgleicht, wobei dieser Ausgleich durch einen thermischen Ausdehnungskoeffizienten erfolgt, dessen Wert zwischen demjenigen des Substrats und der Beschichtung (2) liegt und/oder dadurch erfolgt, daß die Zwischenschicht (4) eine geringere Dichte und einen wesentlich geringeren E-Modul als die der angrenzenden Werkstoffe aufweist.

10. Implantat nach Anspruch 9, **dadurch gekennzeichnet,** daß die Zwischenschicht (4) aus Graphit, Glaskohlenstoff oder pyrolytischem Kohlenstoff besteht, wobei insbesondere bei einem metallischen Substrat die Zwischenschicht (4) eine Legierung der Materialien von Beschichtung (2) und Substrat bildet.

## Claims

1. Implant, in particular for cardiovascular application, with a coating (2) which consists of one of the semiconducting materials silicon carbide (a-SiC:H), silicon nitride (a-SiN:H) or silicon oxide (a-SiO:H), **characterised in that** for the stabilization of the silicate network a slight doping of the coating (2) with phosphorus is provided, in particular in the form of phosphine with a concentration of less than one per cent.

2. Implant according to claim 1, **characterised in that** the semiconducting material is amorphous.

3. Implant according to claim 1, **characterised in that** the semiconducting material is microcrystalline.

4. Implant according to any one of the preceding claims, **characterised in that** the coating (2) has a thickness in the range of 1 µm, at most 10 µm.

5. Implant according to claim 4, **characterised in that** the surface roughness lies in the range of 0.1 µm.

6. Implant according to any one of the preceding claims, **characterised in that** the state density of the coating (2) comes to 10¹⁸ cm⁻³ev⁻¹ or less, so that direct charge exchange processes between coagulation-specific proteins and the implant surface (3) are prevented.

7. Implant according to claim 2, **c haracterised in that** the amorphous silicon carbide (a-SiC:H) is in particular a precipitation product of a mixture containing silane (SiH4) and/or methane (CH4).

8. Implant according to any one of the preceding claims, **characterised in that** the coating (2) is substantially non-porous.

9. Implant according to any one of the preceding claims, **characterised in that** between substrate and coating (2) an intermediate layer (4) is provided which balances out thermal expansions, said balancing out taking place due to a thermal expansion coefficient whose value lies between that of the substrate and of the coating (2) and/or takes place due to that fact that the intermediate layer (4) has a smaller density and a considerably smaller modulus of elasticity than that of the neighbouring materials.

10. Implant according to claim 9, **characterised in that** the intermediate layer (4) consists of graphite, glassy carbon or pyrolytic carbon, the intermediate layer (4) forming particularly in the case of a metallic substrate an alloy of the materials of coating (2) and substrate.

## Revendications

1. Implant, en particulier pour utilisation cardio-vasculaire, comprenant un revêtement (2) qui est constitué de l'un des matériaux semi-conducteurs comprenant le carbure de silicium (a-SiC:H), le nitrure de silicium (a-SiN:H) ou l'oxyde de silicium (a-SiO:H), caractérisé en ce que pour la stabilisation du réseau de silicate, est prévu un dopage léger du revêtement (2) avec du phosphore, en particulier sous la forme de phosphine avec une concentration de moins de 1%.

2. Implant selon la revendication 1, caractérisé en ce que le matériau semi-conducteur est amorphe.

3. Implant selon la revendication 1, caractérisé en ce que le matériau semi-conducteur est micro-cristallin.

4. Implant selon l'une des revendications précédentes, caractérisé en ce que le revêtement (2) a une épaisseur dans la plage allant jusqu'à 1 µm, au maximum 10 µm.

5. Implant selon la revendication 4, caractérisé en ce que la rugosité de surface est située dans la région de 0,1 µm.

6. Implant selon l'une des revendications précédentes, caractérisé en ce que la densité des états du revêtement (2) est de l'ordre de 10¹⁸cm⁻³eV⁻¹ ou moins, de sorte que l'on empêche les processus directs d'échanges de charge entre les protéines spécifiques à la coagulation et la surface de l'implant (3).

7. Implant selon la revendication 2, caractérisé en ce que le carbure de silicium amorphe (a-SiC:H) est en particulier un produit de séparation d'un mélange qui contient du silane (SiH₄) et/ou du méthane (CH₄).

8. Implant selon l'une des revendications précédentes, caractérisé en ce que le revêtement (2) est essentiellement exempt de pores.

9. Implant selon l'une des revendications précédentes, caractérisé en ce qu'une couche intermédiaire (4) est prévue entre le substrat et le revêtement (2), laquelle compense les dilations thermiques, cette compensation ayant lieu grâce à un coefficient de dilatation thermique dont la valeur est située entre celui du substrat et celui du revêtement (2) et/ou par le fait que la couche intermédiaire (4) a une densité inférieure et un module d'élasticité sensiblement inférieur à celui des matériaux adjacents.

10. Implant selon la revendication 9, caractérisé en ce que la couche intermédiaire (4) est réalisée en graphite, en carbone vitreux ou en carbone pyrolytique, et en particulier dans le cas d'un substrat métallique, que la couche intermédiaire (4) forme un alliage des matériaux du revêtement (2) et du substrat.
